(19) Europäisches Patentamt
European Patent Office
Office européen des brevets

(11) **EP 4 099 335 A1**

(12) **EUROPEAN PATENT APPLICATION**

(43) Date of publication:
**07.12.2022 Bulletin 2022/49**

(21) Application number: **22176924.3**

(22) Date of filing: **02.06.2022**

(51) International Patent Classification (IPC):
**G16H 10/40** (2018.01)   **G01N 33/48** (2006.01)
**G16H 50/20** (2018.01)   **G16H 50/70** (2018.01)

(52) Cooperative Patent Classification (CPC):
**G16H 10/40; G16H 50/20; G16H 50/70;** C12Q 1/04;
G01N 2800/368

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **03.06.2021 IN 202121024679**

(71) Applicant: **Tata Consultancy Services Limited Maharashtra (IN)**

(72) Inventors:
• **SINGH, Rohan**
  **411013 Pune - Maharashtra (IN)**
• **NAGPAL, Sunil**
  **411013 Pune - Maharashtra (IN)**

• **SRIVASTAVA, Divyanshu**
  **411013 Pune - Maharashtra (IN)**
• **HAQUE, Mohammed Monzoorul**
  **411013 Pune - Maharashtra (IN)**
• **MANDE, Sharmila Shekhar**
  **411013 Pune - Maharashtra (IN)**

(74) Representative: **Goddar, Heinz J.**
**Boehmert & Boehmert**
**Anwaltspartnerschaft mbB**
**Pettenkoferstrasse 22**
**80336 München (DE)**

Remarks:
The complete document including Reference Table(s) and the Sequence Listing(s) can be downloaded from the EPO website

(54) **SYSTEM AND METHOD FOR ESTIMATION OF DELIVERY DATE OF PREGNANT SUBJECT USING MICROBIOME DATA**

(57)      The need for an accurate, early, and precise estimation of expected delivery date (EDD) for the pregnant subject is vital. A system and method for predicting a day/date of delivery for a pregnant subject using one or more microbiome samples collected from the pregnant subject is provided. The disclosure relates to applying machine learning techniques on the microbiome characterization data corresponding to the biological sample(s) collected from the pregnant subject. The method further comprises using the predicted EDD to suitably plan and take required medical treatment or precautions or medical advice for the pregnant subject to prevent any pregnancy and/or delivery related complications and to manage the delivery appropriately. The disclosure also provides compositions of the microbiome data which can potentially influence the delivery date, or the method provides exemplary compositions of the microbiome data which plays vital role in estimating the EDD of the pregnant subject.

FIG. 1

**Description**

CROSS-REFERENCE TO RELATED APPLICATIONS AND PRIORITY

The present application claims priority from Indian provisional application no.202121024679, filed on June 03, 2021.

TECHNICAL FIELD

The embodiments herein generally relate to the field of prediction of delivery date of a pregnant subject, more specifically estimation of an expected delivery date (EDD) of a pregnant subject using one or more biological samples collected from the pregnant subject.

BACKGROUND

Reproduction to carry ones' lineage forward is a deeply wired trait in all living organisms, including humans. The childbearing gender of different species undergo a phase of gestation involving in situ development of the embryo/ offspring, which subsequently will be delivered after a standard period of gestation, the said gestation period varying in different species. In humans, for example, the standard gestation period is approximately 280 days or 40 weeks. However, in a substantial number of cases, it is observed that a child is born pre-term i.e. any time before 259 days i.e. 37 completed weeks of gestation, or in case of the 'term' deliveries, the child is born at different time periods in different subjects i.e. ranging anytime between 259 to 280 days of gestation or in some cases, even more than that.

The preterm can be classified into varying buckets based on the time of delivery happening before 37 weeks of completed gestation as given below:

    1) extreme preterm (<28 weeks of gestation)
    2) very preterm (28 to 32 weeks of gestation)
    3) moderate or late preterm (32 to <37 completed weeks of gestation).

Considering such variations in the expected period of gestation and observed delivery date outcomes, a prior prediction about an estimated day/date of delivery is extremely useful for taking several medical related decisions including but not limited to mode of pregnancy management or type of treatment required if any and the like. It also has extreme importance with respect to social, financial, and emotional aspects of the pregnant subject and her family/ acquaintances. It is also essential for the medical care givers and associated clinics/ hospitals to suitably plan aspects pertaining to arrangement and reservation of emergency healthcare services (for both predicted as well as for unforeseen events) well ahead in time.

It is observed that infants born extreme preterm (less than 28 weeks of gestation) and very preterm (28 to 32 weeks) are at the highest risk of mortality, whereas infants who are born at moderate (Gestational age between 32 weeks and 33 weeks and 6 days) or late preterm (34 and almost 37 weeks of pregnancy) experience greater complications than infants born at term delivery. This is a serious threat to the health of the infants as well as the health of the mother. Further, preterm deliveries pose enormous logistic, infrastructure, and medical challenges given that procedures like Caesarean sections are sometimes needed to be performed on the pregnant subject on short notice. Given that most preterm babies are admitted to neonatal intensive care unit (NICU), preterm birth prognosis in terms of anticipated day/ week of preterm delivery becomes a crucial aspect for planning/ availing usage of neonatal intensive care units (NICUs).

The need for an accurate, early, and precise estimation of expected delivery date (EDD) for the pregnant subject is vital because many times delays in prediction or misguidance arising from imprecise EDD estimation may translate to unnecessary medically-assisted pre-induction of labour, needless/ premature caesareans, increasing medical costs, emotional stress, logistic burden on healthcare services and providers, and impact on families of the prospective parents and among others.

Currently, ultrasound imaging (USG) has been classically employed for diagnosing CNS (central nervous system) malformations (if any) in the foetus and also used for estimating the day of delivery (EDD) of pregnant subject i.e. delivery/ birth day of the unborn foetus by taking measurements of foetus cranium, abdomen and/ or the femur bone. These measurements are further analyzed using empirically derived regression models based on the observed population-level characteristics to predict EDD and/or foetus malformations (if any). However, this existing technique has certain drawbacks. The accuracy of the measurements taken using USG post 24 weeks of gestation is dependent on factors such as USG operator expertise, approximation errors due to foetus abnormalities, inconsistencies stemming from skull size approximation, and subjectivity in 2D diagnostic plane finding. As there are multiple approximation errors, these errors directly affect the estimation of expected delivery date. Generally, advanced (or later) stages of pregnancy are characterized with an increase in biological variations in normal foetuses, and hence USG based measurements and predictions performed using such measurements data in the later stages of pregnancy that is in the 2nd or 3rd trimesters of pregnancy are prone to higher error margins with respect to the estimated date of delivery.

Furthermore, in some cases, ultrasound imaging facility may not be affordable or available to the family. Importantly, in the existing methods and technologies, estimation of delivery date is focused mostly in isolation on observable abnormalities in the unborn child/foetus or abnormalities or symptoms in pregnant subject, rather than probing into the inherent global features such as biochemical, microbial, and physiological profiles of the pregnant subject.

Further, the existing methods of pregnancy duration related predictions/estimations are more inclined towards developing and using empirically derived models for predicting term vs pre-term birth outcomes of pregnancy rather than precisely predicting the day/ date of delivery. The existing methods for estimating day/ date of delivery are highly restricted given that they are applicable more during the first or second trimesters of pregnancy and additionally suffer from lower accuracies. It may be noted that in conventional techniques, more attention given to classifying the pregnancy into term or pre-term with the limited resolution at the level of the day of delivery. Moreover, these existing techniques rely more on procedures that are invasive in nature. Moreover, given that conventional techniques e.g. USG or biochemical tests are merely providing predictions about the delivery date outcome in the form of term or preterm birth, and they fail to provide any precise delivery day/ date of the pregnant subject.

Another conventional techniques discloses application of machine learning technique in gestational age prediction. This technique is based on ultrasound scans of 'foetal brain' as a feature-based machine learning model for gestational delivery prediction. This technique is specifically focused on scanning in 2nd and 3rd trimester of pregnancy. This technique however does not consider important parameters pertaining to the pregnant mother, especially overall microbiome profile of the pregnant subject. One of the prior art techniques, use of cell-free RNA (cfRNA) transcripts from 'foetal tissues' in maternal blood are used as features for generating a machine learning model for predicting the pre-term outcome of delivery. However, the mentioned microbiome/ transcriptome models merely attempt to classify the outcome of pregnancy into term and pre-term, that too in first trimester alone, which remains a major limitation for any model in the current state of the art.

Therefore, the existing techniques focused on providing predictions related to pregnancy delivery date are merely directed to predicting either term or preterm delivery and lack in providing accurate predictions with respect to estimated delivery day/ date for the pregnant subject.

SUMMARY

Embodiments of the present disclosure present technological improvements as solutions to one or more of the above-mentioned technical problems recognized by the inventors in conventional systems. For example, in one embodiment, a method for estimation of an expected delivery date (EDD) of a pregnant subject using microbiome data is provided. Initially, a set of biological samples is collected on a predefined set of days from the pregnant subject whose EDD is to be estimated. Further, microbiome profiling is performed for each of the collected biological samples to obtain a microbiome profile data comprising of a plurality of metagenomic features. In the next step, a set of expected delivery dates is predicted for the pregnant subject by providing the microbiome profile data as input to a set of models present in a prebuilt ensemble. And finally, a central tendency value of the predicted set of expected delivery dates is calculated to determine the EDD of the pregnant subject, wherein the predefined set of days are pre-ascertained, and the ensemble is built using the steps of: collecting biological samples from a plurality of pregnant subjects at predefined time points during a period of pregnancy; performing microbiome profiling for each of the collected biological samples to obtain a microbiome profile data comprising of the plurality of metagenomic features; training one or a combination of regressors using the obtained microbiome profile data; reconstructing, using the trained regressors, for each pregnant subject among the plurality of pregnant subjects, a simulated microbiome profile, wherein the simulated microbiome profile comprises of values in original microbiome profile data and reconstructed values of all metagenomic features for specific days of the pregnancy duration when biological samples were not collected from the pregnant subject; constructing, using the simulated microbiome profile, a multi-variate matrix for each of the metagenomic features, wherein the multi-variate matrix refers to the microbiome profile of the metagenomic feature observed in each of the pregnant subjects across entire duration of pregnancy, wherein each matrix comprises a unique participant ID corresponding to each pregnant subject in rows and each day of pregnancy, in which the microbiome profile of the metagenomic feature is observed, as columns; training a regressor model with a k-fold cross validation on a predefined subset of rows in the multi-variate matrix for each metagenomic feature, wherein values of the metagenomic feature across the entire pregnancy duration belonging to the subset of the multi-variate matrix forms independent variables and corresponding date of delivery forms the target variable; computing prediction errors of the regressor models generated during k-folds cross validation performed on the subset of the multi-variate matrix; repeating the step of training the regressor model with k-fold cross validation for a particular metagenomic feature, each time using input data corresponding to one of a plurality of pregnancy windows, wherein each pregnancy window corresponds to a pregnancy duration that is progressively lesser by one week as compared to the previous or the next pregnancy window; computing corresponding prediction errors for input data corresponding to each pregnancy window; identifying, for each metagenomic feature, a predefined number of top pregnancy windows which resulted in the smallest prediction errors; selecting, from amongst identified predefined number of top pregnancy windows, a pregnancy window that occurs the maximum number of times across all metagenomic features; retrieving, for each metagenomic feature, the respective regressor model corresponding to the selected pregnancy window the regressor model comprises a plurality of temporal features; sorting the plurality of temporal features of the regressor model based on their importance scores and selecting, a set of top scoring temporal features, wherein the selected set of temporal features refer to a specific set of days within the selected pregnancy window; repeating the steps of retrieving and sorting for each of the metagenomic features in microbiome profile data; identifying and selecting, from amongst the specific set of days occurring in the selected pregnancy window, a predefined number of days that are associated to the metagenomic features; generating, for each metagenomic feature, a feature-specific regressor model with a k-fold cross validation on a custom multi-variate matrix comprising of data pertaining to the numeric values of the features on the identified specific subset of days; selecting a subset of models out of the models trained, wherein the selected models have the lowest prediction error; and forming an ensemble using the selected subset of models.

In another aspect the method also comprises planning and managing healthcare emergency services in a healthcare unit using the expected delivery date of pregnant subject. The method comprises computing 'weekly predicted emergency burden (wPEB)' metrics associated with the healthcare unit which are critical to handle the healthcare emergency, based on the registered pregnant subject's information submitted to the healthcare unit.

In another aspect, a system for estimation of an expected delivery date (EDD) of a pregnant subject using microbiome data is provided. The system comprises a microbiome sample receiver, a microbiome characterization platform, one or more hardware processors and a memory. The microbiome sample receiver collects a set of biological samples on a predefined set of days from the pregnant subject whose EDD is to be estimated. The microbiome characterization platform performs microbiome profiling for each of the collected biological samples to obtain a microbiome profile data comprising of a plurality of metagenomic features. The memory is in communication with the one or more hardware processors, wherein the one or more first hardware processors are configured to execute programmed instructions stored in the one or more first memories, to: predict a set of expected delivery dates for the pregnant subject by providing the microbiome profile data as input to a set of models present in a prebuilt ensemble; and calculate a central tendency value of the predicted set of expected delivery dates to determine the EDD of the pregnant subject, wherein the predefined set of days are pre-ascertained, and the ensemble is built using the steps of: collecting biological samples from a plurality of pregnant subjects at predefined time points during a period of pregnancy; performing microbiome profiling for each of the collected biological samples to obtain a microbiome profile data comprising of the plurality of metagenomic features; training one or a combination of regressors using the obtained microbiome profile data; reconstructing, using the trained regressors, for each pregnant subject among the plurality of pregnant subjects, a simulated microbiome profile, wherein the simulated microbiome profile comprises of values in original microbiome profile data and reconstructed values of all metagenomic features for specific days of the pregnancy duration when biological samples were not collected from the pregnant subject; constructing, using the simulated microbiome profile, a multi-variate matrix for each of the metagenomic features, wherein the multi-variate matrix refers to the microbiome profile of the metagenomic feature observed in each of the pregnant subjects across entire duration of pregnancy, wherein each matrix comprises a unique participant ID corresponding to each pregnant subject in rows and each day of pregnancy, in which the microbiome profile of the metagenomic feature is observed, as columns; training a regressor model with a k-fold cross validation on a predefined subset of rows in the multi-variate matrix for each metagenomic feature, wherein values of the metagenomic feature across the entire pregnancy duration belonging to the subset of the multi-variate matrix forms independent variables and corresponding date of delivery forms the target variable; computing prediction errors of the regressor models generated during k-folds cross validation performed on the subset of the multi-variate matrix; repeating the step of training the regressor model with k-fold cross validation for a particular metagenomic feature, each time using input data corresponding to one of a plurality of pregnancy windows, wherein each pregnancy window corresponds to a pregnancy duration that is progressively lesser by one week as compared to the previous or the next pregnancy window; computing corresponding prediction errors for input data corresponding to each pregnancy window; identifying, for each metagenomic feature, a predefined number of top pregnancy windows which resulted in the smallest prediction errors; selecting, from amongst identified predefined number of top pregnancy windows, a pregnancy window that occurs the maximum number of times across all metagenomic features; retrieving, for each metagenomic feature, the respective regressor model corresponding to the selected pregnancy window the regressor model comprises a plurality of temporal features; sorting the plurality of temporal features of the regressor model based on their importance scores and selecting, a set of top scoring temporal features, wherein the selected set of temporal features refer to a specific set of days within the selected pregnancy window; repeating the steps of retrieving and sorting for each of the metagenomic features in microbiome profile data; identifying and selecting, from amongst the specific set of days occurring in the selected pregnancy window, a predefined number of days that are associated to the metagenomic features; generating, for each metagenomic feature, a feature-specific regressor model with a k-fold cross validation on a custom multi-variate matrix comprising of data pertaining to the numeric values of the features on the identified specific subset of days; selecting a subset of models out of the models trained, wherein the selected models have the lowest prediction error; and forming an ensemble using the selected subset of models.

In yet another aspect, one or more non-transitory machine-readable information storage mediums comprising one or more instructions which when executed by one or more hardware processors cause estimation of an expected delivery date (EDD) of a pregnant subject using microbiome data. Initially, a set of biological samples is collected on a predefined set of days from the pregnant subject whose EDD is to be estimated. Further, microbiome profiling is performed for each of the collected biological samples to obtain a microbiome profile data comprising of a plurality of metagenomic features. In the next step, a set of expected delivery dates is predicted for the pregnant subject by providing the microbiome profile data as input to a set of models present in a prebuilt ensemble. And finally, a central tendency value of the predicted set of expected delivery dates is calculated to determine the EDD of the pregnant subject, wherein the predefined set of days are pre-ascertained, and the ensemble is built using the steps of: collecting biological samples from a plurality of pregnant subjects at predefined time points during a period of pregnancy; performing microbiome profiling for each of the collected biological samples to obtain a microbiome profile data comprising of the plurality of metagenomic features; training one or a combination of regressors using the obtained microbiome profile data; reconstructing, using the trained regressors, for each pregnant subject among the plurality of pregnant subjects, a simulated microbiome profile, wherein the simulated microbiome profile comprises of values in original microbiome profile data and reconstructed values of all metagenomic features for specific days of the pregnancy duration when biological samples were not collected from the pregnant subject; constructing, using the simulated microbiome profile, a multi-variate matrix for each of the metagenomic features, wherein the multi-variate matrix refers to the microbiome profile of the metagenomic feature observed in each of the pregnant subjects across entire duration of pregnancy, wherein each matrix comprises a unique participant ID corresponding to each pregnant subject in rows and each day of pregnancy, in which the microbiome profile of the metagenomic feature is observed, as columns; training a regressor model with a k-fold cross validation on a predefined subset of rows in the multi-variate matrix for each metagenomic feature, wherein values of the metagenomic feature across the entire pregnancy duration belonging to the subset of the multi-variate matrix forms independent variables and corresponding date of delivery forms the target variable; computing prediction errors of the regressor models generated during k-folds cross validation performed on the subset of the multi-variate matrix; repeating the step of training the regressor model with k-fold cross validation for a particular metagenomic feature, each time using input data corresponding to one of a plurality of pregnancy windows, wherein each pregnancy window corresponds to a pregnancy duration that is progressively lesser by one week as compared to the previous or the next pregnancy window; computing corresponding prediction errors for input data corresponding to each pregnancy window; identifying, for each metagenomic feature, a predefined number of top pregnancy windows which resulted in the smallest prediction errors; selecting, from amongst identified predefined number of top pregnancy windows, a pregnancy window that occurs the maximum number of times across all metagenomic features; retrieving, for each metagenomic feature, the respective regressor model corresponding to the selected pregnancy window the regressor model comprises a plurality of temporal features; sorting the plurality of temporal features of the regressor model based on their importance scores and selecting, a set of top scoring temporal features, wherein the selected set of temporal features refer to a specific set of days within the selected pregnancy window; repeating the steps of retrieving and sorting for each of the metagenomic features in microbiome profile data; identifying and selecting, from amongst the specific set of days occurring in the selected pregnancy window, a predefined number of days that are associated to the metagenomic features; generating, for each metagenomic feature, a feature-specific regressor model with a k-fold cross validation on a custom multi-variate matrix comprising of data pertaining to the numeric values of the features on the identified specific subset of days; selecting a subset of models out of the models trained, wherein the selected models have the lowest prediction error; and forming an ensemble using the selected subset of models.

It is to be understood that both the foregoing general description and the following detailed description are exemplary and explanatory only and are not restrictive of the invention, as claimed.

BRIEF DESCRIPTION OF THE DRAWINGS

The accompanying drawings, which are incorporated in and constitute a part of this disclosure, illustrate exemplary embodiments and, together with the description, serve to explain the disclosed principles:

FIG. 1 illustrates a block diagram of a system for estimation of an expected delivery date (EDD) of a pregnant subject using microbiome data according to some embodiments of the present disclosure.
FIG. 2 shows a flowchart illustrating the steps involved in a method for estimation of an expected delivery date (EDD) of a pregnant subject using microbiome data according to some embodiments of the disclosure.
FIG. 3A-3B shows a flowchart illustrating steps involved in building an ensemble to be used in system of FIG. 1 according to some embodiments of the disclosure.

## DETAILED DESCRIPTION OF EMBODIMENTS

Exemplary embodiments are described with reference to the accompanying drawings. Wherever convenient, the same reference numbers are used throughout the drawings to refer to the same or like parts. While examples and features of disclosed principles are described herein, modifications, adaptations, and other implementations are possible without departing from the scope of the disclosed embodiments.

The need for an accurate, early, and precise estimation of expected delivery date (EDD) for the pregnant subject is vital because many times delays in prediction or misguidance arising from imprecise EDD estimation may translate to unnecessary medically-assisted pre-induction of labour, needless/ premature caesareans, increasing medical costs, emotional stress, logistic burden on healthcare services and providers, and impact on families of the prospective parents and among others.

Currently, ultrasound imaging (USG) has been classically employed for diagnosing CNS (central nervous system) malformations (if any) in the foetus and also used for estimating the day of delivery (EDD) of pregnant subject i.e. delivery/ birth day of the unborn foetus by taking measurements of foetus cranium, abdomen and/ or the femur bone. These measurements are further analyzed using empirically derived regression models based on the observed population-level characteristics to predict EDD and/or foetus malformations (if any). However, this existing technique has certain drawbacks. The accuracy of the measurements taken using USG post 24 weeks of gestation is dependent on factors such as USG operator expertise, approximation errors due to foetus abnormalities, inconsistencies stemming from skull size approximation, and subjectivity in 2D diagnostic plane finding. As there are multiple approximation errors, these errors directly affect the estimation of expected delivery date. Generally, advanced (or later) stages of pregnancy are characterized with an increase in biological variations in normal foetuses, and hence USG based measurements and predictions performed using such measurements data in the later stages of pregnancy that is in the 2nd or 3rd trimesters of pregnancy are prone to higher error margins with respect to the estimated date of delivery.

Furthermore, in some cases, ultrasound imaging facility may not be affordable or available to the family. Importantly, in the existing methods and technologies, estimation of delivery date is focused mostly in isolation on observable abnormalities in the unborn child/foetus or abnormalities or symptoms in pregnant subject, rather than probing into the inherent global features such as biochemical, microbial, and physiological profiles of the pregnant subject.

Further, the existing methods of pregnancy duration related predictions/estimations are more inclined towards developing and using empirically derived models for predicting term vs pre-term birth outcomes of pregnancy rather than precisely predicting the day/ date of delivery. The existing methods for estimating day/ date of delivery are highly restricted given that they are applicable more during the first or second trimesters of pregnancy and additionally suffer from lower accuracies. It may be noted that in conventional techniques, more attention given to classifying the pregnancy into term or pre-term with the limited resolution at the level of the day of delivery. Moreover, these existing techniques rely more on procedures that are invasive in nature. Moreover, given that conventional techniques e.g. USG or biochemical tests are merely providing predictions about the delivery date outcome in the form of term or preterm birth, and they fail to provide any precise delivery day/ date of the pregnant subject.

The present disclosure provides a system and a method for predicting a day/date of delivery for a pregnant subject using one or more microbiome samples collected from the pregnant subject. The disclosure provides a method and system for estimation of an expected delivery date (EDD) of the pregnant subject using microbiome data. It should be appreciated that the terms 'expected delivery date' or 'estimated delivery date' or 'EDD' are interchangeable in the present disclosure'. More particularly, the disclosure herein relates to applying one or more machine learning techniques on the microbiome characterization data corresponding to the biological sample(s) collected from the pregnant subject, to predict the expected delivery date (EDD of the pregnant subject, wherein the microbiome characterization data (optionally) coupled with other subject specific data/ metadata (e.g. age, race, biochemistry or metabolic profile, etc., of the pregnant subject). The method further comprises using the predicted EDD to suitably plan and take required medical treatment or precautions or medical advice for the pregnant subject to prevent any pregnancy and/or delivery related complications and/or to manage the delivery appropriately.

Further, presently disclosed method also provides exemplary compositions of the microbiome data which can potentially influence the delivery date of the pregnant subject or the method provides exemplary compositions of the microbiome data which are playing a vital role in predicting/estimating the delivery date of the pregnant subject. Therefore, the method uses the so disclosed compositions of the microbiome data of the pregnant subject as an input for the regression models to predict the delivery date with improved accuracy and reliability.

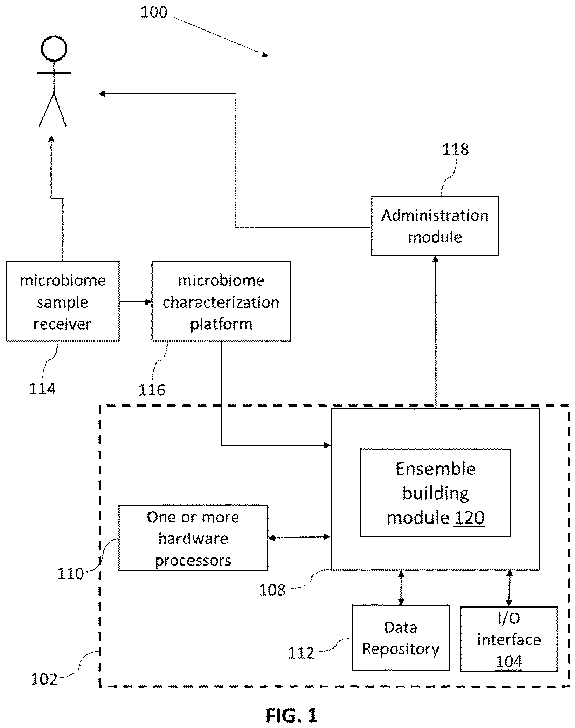

Referring now to the drawings, and more particularly to FIG. 1 through FIG. 3B, where similar reference characters denote corresponding features consistently throughout the figures, there are shown preferred embodiments and these embodiments are described in the context of the following exemplary system and/or method.

FIG.1 illustrates a block diagram of a system 100 for estimating a date of delivery for a pregnant subject using one or more microbiome samples collected from the pregnant subject, according to some embodiments of the present disclosure. The microbiome samples are collected at a scheduled / specific time points during the pregnancy period of the pregnant subject.

The estimated day/ date of delivery for the pregnant subject is the estimated due date i.e. the day/ date of birth of unborn child at the end of pregnancy or gestation period of the pregnant subject. In one of the aspects, the pregnant subject is a pregnant woman or a female viviparous animal. The disclosure also serves as a surrogate or a companion screening method that enables/ complements other existing methods used for predicting, at any time point in pregnancy, the expected day/ date of delivery of the unborn child of the pregnant woman. The presently disclosed method for predicting the day of delivery of a pregnant subject comprises using microbiome characterization data corresponding to the pregnant subject along with secondary data including at least one of microbiome data, or microbiome-associated properties e.g. bacterial/microbial abundance data, bacterial/microbial community diversity data, bacterial/microbial proteins, bacterial/microbial metabolites, bacterial/microbial metabolic pathways, in conjugation with biological and microbial features (jointly referred as metagenomic features) of the pregnant subject, and features of an unborn child (foetus). The biological features of the unborn child (foetus) may comprise gestational age of the unborn child / foetus, blood biochemistry of the unborn child or foetus.

The system 100 is configured to generate microbial features or metagenomic features corresponding to the pregnant subject and providing those metagenomic features of the pregnant subject as input to supervised machine learning techniques to predict delivery day/date of the pregnant subject. The microbial features of the pregnant subject comprise but not limited to microbial genus-level abundance profile, microbial family-level abundance profile, microbial sequence cluster profile and microbial diversity profile which are obtained using the biological/microbiome sample of the pregnant subject. The biological features of the pregnant subject comprise the biological features including biochemistry, physiology and/ or age-related features of the pregnant subject. The biological features of the unborn child (foetus) may comprise age of the unborn child / foetus, blood biochemistry of the unborn child or foetus which can be used along with the biological and microbial features of the pregnant subject for prediction of day of delivery of the pregnant subject.

The method also comprises identifying the most suitable sub-set of machine learning models to process the metagenomic features data of the pregnant subject to predict the day/ date of delivery. The supervised machine learning models that can be used in the present technique comprise but are not limited to regressors/ regression models comprising linear regression models, decision tree regressors, boosted regression models, random forest regression models, and the like. The method further comprises employing machine learning classification techniques (e.g. random forest classifier, logistic regression, KNN, Support Vector Machine, Naive Bayes) for identifying the most appropriate combination of regression models which can accurately and reliably predict delivery date of the pregnant subject.

According to the present embodiment, generalized microbiome feature based machine learning models are generated using the microbiome data of the pregnant subject. These generalized microbiome features based machine learning models add significant value by way of helping in stratifying cases of preterm delivery.

The above-mentioned precise classification of preterm delivery of the pregnant subject further enables administration of more specific and streamlined medications to the pregnant subject or related to the foetus/unborn child, if required. The generalized microbiome features based models can predict delivery date for any pregnancy outcome including term or preterm, with reliable accuracy in predicting dates for normal, adverse, very adverse, potentially fatal outcomes and even delayed outcomes. The method specifically uses microbiome features of pregnant subject in combination with other biological features/parameters of the pregnant subject as well as of the foetus which leads to improved accuracy and reliability in estimation of delivery date for the pregnant subject.

According to present embodiment of the disclosure, the method further provides creation of digital machine deployable model(s) for mobile applications, websites enabling automation of a predictive system to automatically predict delivery date for the pregnant subject at any given time point in pregnancy i.e. in any of the three trimesters of pregnancy including first to late third trimester of pregnancy.

Although the present disclosure is explained considering that the system 100 is implemented on a server, it may also be present elsewhere such as a local machine. It may be understood that the system 100 comprises one or more computing devices 102, such as a laptop computer, a desktop computer, a notebook, a workstation, a cloud-based computing environment and the like. It will be understood that the system 100 may be accessed through one or more input/output interface 104. Examples of the I/O interface 104 may include, but are not limited to, a user interface, a portable computer, a personal digital assistant, a handheld device, a smartphone, a tablet computer, a workstation and the like. The I/O interface 104 are communicatively coupled to the system 100 through a network (not shown in Fig.).

In an embodiment, the network may be a wireless or a wired network, or a combination thereof. In an example, the network can be implemented as a computer network, as one of the different types of networks, such as virtual private network (VPN), intranet, local area network (LAN), wide area network (WAN), the internet, and such. The network may either be a dedicated network or a shared network, which represents an association of the different types of networks that use a variety of protocols, for example, Hypertext Transfer Protocol (HTTP), Transmission Control Protocol/Internet Protocol (TCP/IP), and Wireless Application Protocol (WAP), to communicate with each other. Further, the network may include a variety of network devices, including routers, bridges, servers, computing devices, storage devices. The network devices within the network may interact with the system 100 through communication links.

The system 100 may be implemented in a workstation, a mainframe computer, a server, and a network server. In an embodiment, the computing device 102 further comprises one or more hardware processors 110, one or more memory 108, hereinafter referred as a memory 108 and a data repository 112, for example, a repository 112. The memory 108 is in communication with the one or more hardware processors 110, wherein the one or more hardware processors 110 are configured to execute programmed instructions stored in the memory 108, to perform various functions as explained in the later part of the disclosure. The repository 112 may store data processed, received, and generated by the system 100.

The system 100 supports various connectivity options such as BLUETOOTH®, USB, ZigBee and other cellular services. The network environment enables connection of various components of the system 100 using any communication link including Internet, WAN, MAN, and so on. In an exemplary embodiment, the system 100 is implemented to operate as a stand-alone device. In another embodiment, the system 100 may be implemented to work as a loosely coupled device to a smart computing environment. The components and functionalities of the system 100 are described further in detail.

According to an embodiment of the disclosure, the system 100 consists of a microbiome sample receiver 114 and a microbiome characterization platform 116, and an administration module 118 as shown in FIG. 1. The processor 110 is in communication with the memory 108. The memory 108 further includes a plurality of modules for performing various functions. The plurality of modules comprises an ensemble building module 120.

The microbiome sample receiver 114 receives the microbiome sample from the pregnant subject. The microbiome characterization platform 116 comprises at least one of the sequencing platform, a microscopy platform, a nucleic acid hybridization platform, internet-enabled qPCR (Quantitative Polymerize Chain Reaction) machine or a cell sorting platform to obtain/generate the microbiome characterization data from the microbiome sample.

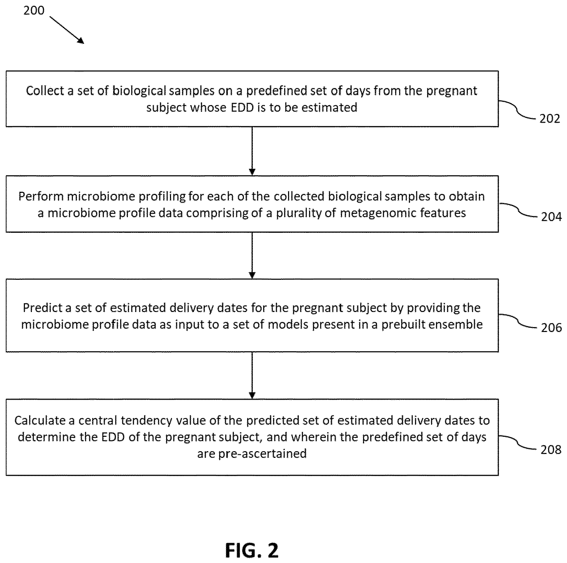

FIG. 2 show an exemplary flow diagram illustrating a method 200 for estimation of the expected delivery date (EDD) of the pregnant subject using microbiome data according to some embodiments of the present disclosure. The method 200 depicted in the flow chart may be executed by a system, for example, the system 100 of FIG. 1. In an example embodiment, the system 100 may be embodied in a computing device.

Operations of the flowchart, and combinations of operations in the flowchart, may be implemented by various means, such as hardware, firmware, processor, circuitry and/or other device associated with execution of software including one or more computer program instructions. For example, one or more of the procedures described in various embodiments may be embodied by computer program instructions. In an example embodiment, the computer program instructions, which embody the procedures, described in various embodiments may be stored by at least one memory device of a system and executed by at least one processor in the system. Any such computer program instructions may be loaded onto a computer or other programmable system (for example, hardware) to produce a machine, such that the resulting computer or other programmable system embody means for implementing the operations specified in the flowchart. It will be noted herein that the operations of the method 200 are described with help of system 100. However, the operations of the method 200 can be described and/or practiced by using any other system.

At step 202 of the method 200, the system 100 is configured to collect a set of biological samples on a predefined set of days from the pregnant subject whose EDD is to be estimated. The pregnant subject is a pregnant woman, or the pregnant subject is a female viviparous animal.

In one implementation, the microbiome sample receiver 114 may collect or receive the microbiome sample from the pregnant subject. The microbiome sample may be obtained from a mouth, skin, a gut, a vagina or other body sites of the pregnant subject. The microbiome sample is selected from a group comprising a vaginal swab sample, a cervical mucus sample, a cervical swab sample, a vaginal swab including swab sample of a fornix, a urine sample, an amniotic fluid sample, a blood sample , a serum sample, a plasma sample, a placental swab, an umbilical swab, a stool sample, a skin swab, an oral swab, a saliva sample, a periodontal swab, a throat swab, a nasal swab, a vesicle fluid sample, a nasopharyngeal swab, a nares swab, a conjunctival swab, a genital swab, a rectum swab, a tracheal aspirate, and a bronchial swab.

At step 204 of the method 200, the system 100 is configured to perform microbiome profiling for each of the collected biological samples to obtain a microbiome profile data comprising of a plurality of metagenomic features. The plurality of metagenomic features comprises one or both of microbial features and host features.

In order to obtain the microbial features from the microbiome sample, the system 100 is configured to process the microbiome sample via the microbiome characterization platforms 116, to obtain microbiome characterization data or microbiome profile data. The microbiome profile data is obtained by applying one or more techniques comprising a sequencing technique, a microscopic examination, a flow cytometry method, an in-vitro culture-based method, one or more enzymatic or fluorescence assays, or one or more assays involving spectroscopic identification and screening of signals from complex microbial populations. The microbiome profile data comprises sequenced microbial DNA data, a Microscopic imaging data, a Flow cytometry cellular measurement data, a colony count, and cellular phenotypic data of microbes grown in in-vitro cultures, and a signal intensity data.

In one of the embodiment, the sequenced microbial DNA data is obtained from the microbiome sample via a sequencing platform. Obtaining the sequenced microbial DNA data comprises isolating microbial DNA corresponding to the plurality of microbes present in the microbiome sample using at least one DNA extraction technique. In one implementation, the sequencing platform isolates the microbial DNA corresponding to the plurality of microbes present in the microbiome sample using at least one DNA extraction technique.

Further, the microbial DNA is sequenced using at least one DNA sequencing technique to obtain the sequenced microbial DNA data. In one implementation, the sequencing platform sequences the microbial DNA using at least one DNA sequencing technique to obtain the sequenced microbial DNA data. In one aspect, bacterial 16S rRNA or 23S rRNA marker genes are amplified and sequenced from the microbial DNA. In another aspect, either a full-length or one or more specific regions of the bacterial 16S rRNA or 23S rRNA marker genes are amplified and sequenced from the microbial DNA. In one implementation, the sequencing platform amplifies and sequences a bacterial 16S rRNA or 23S rRNA marker genes from the microbial DNA so extracted from the microbiome sample.

In one embodiment, one or more phylogenetic marker genes may be amplified and sequenced from the microbial DNA. The DNA sequencing technique may be selected from a next-generation sequencing techniques, wherein the next-generation sequencing techniques comprises a Whole Genome Shotgun (WGS) sequencing, a fragment library based sequencing technique, a mate-pair library or a paired-end library based sequencing technique, or a combination thereof.

At step 206 of the method 200, the system 100 is configured to predict a set of expected delivery dates for the pregnant subject by providing the microbiome profile data as input to a set of models present in a prebuilt ensemble. The steps of building the prebuilt ensemble is explained in the later part of the disclosure.

At step 208 of the method 200, the system 100 is configured to calculate a central tendency value of the predicted set of expected delivery dates to determine the EDD of the pregnant subject. The predefined set of days are pre-ascertained. In an embodiment, the central tendency value is one of a mean or a median of the predicted set of expected delivery dates. Though any other mathematical measures can be used that indicate a representative value of the set of expected delivery dates.

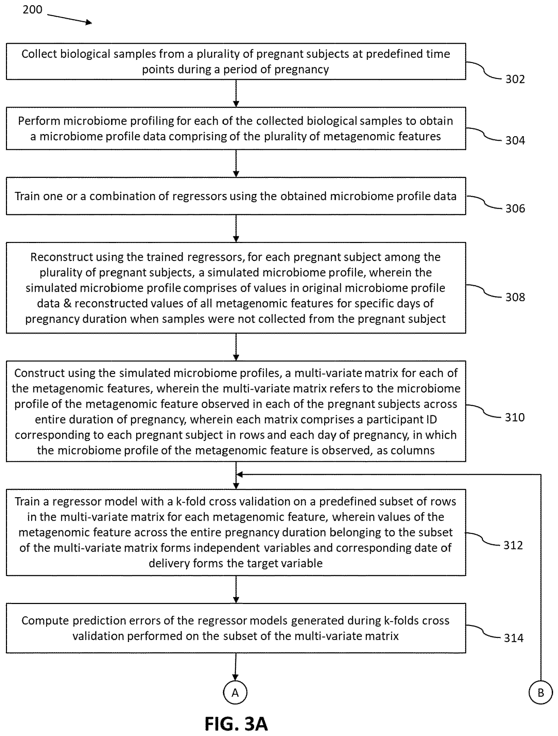

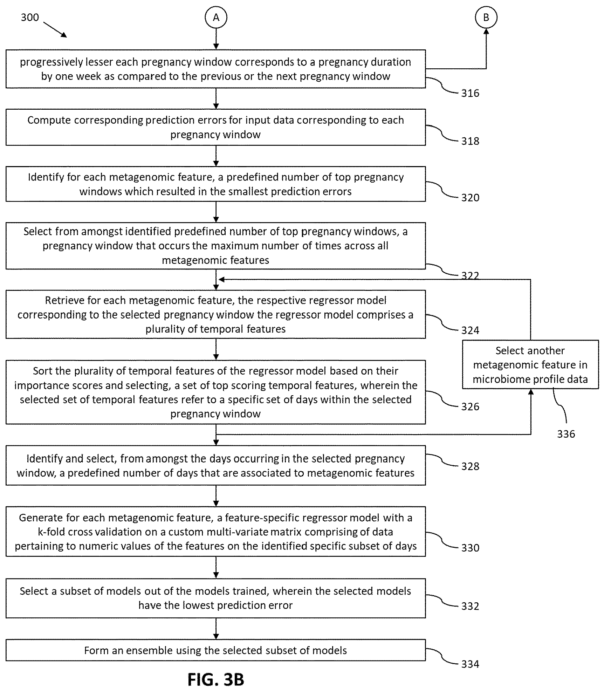

According to an embodiment of the disclosure a flowchart illustrating the steps involved in the building the ensemble is shown in FIG. 3A-3B. Initially at step 302, the biological samples are collected from a plurality of pregnant subjects at predefined time points during a period of pregnancy. It should be appreciated that the biological samples can also be taken from any database.

Further at step 304, microbiome profiling is performed for each of the collected biological samples to obtain the microbiome profile data comprising of the plurality of metagenomic features using the microbiome characterization platform 116. At step 306, one or a combination of regressors are trained using the obtained microbiome profile data.

At step 308, for each pregnant subject among the plurality of pregnant subjects, a simulated microbiome profile is reconstructed using the trained regressors. The simulated microbiome profile comprises of values in the original microbiome profile data and reconstructed values of all metagenomic features for specific days of the pregnancy duration when biological samples were not collected from the pregnant subject.

In another embodiment of the disclosure, different statistical or machine learning algorithms can be further incorporated within the current implementation, such as multivariate iterative imputation which uses other features of the same host to simultaneously regress the missing values of temporal features, or temporal deep learning methods like long-short term memory and recurrent neural networks, or by using a feature-specific pooled data from multiple hosts rather than regressing from the same host.

At step 310, a multi-variate matrix is constructed for each of the metagenomic features using the simulated microbiome profiles, The multi-variate matrix refers to the microbiome profile of the metagenomic feature observed in each of the pregnant subjects across entire duration of pregnancy, wherein each matrix comprises a unique participant ID corresponding to each pregnant subject in rows and each day of pregnancy, in which the microbiome profile of the metagenomic feature is observed, as columns.

At step 312, a regressor model is trained with a k-fold cross validation on a predefined subset of rows in the multi-variate matrix for each metagenomic feature. The values of the metagenomic feature across the entire pregnancy duration belonging to the subset of the multi-variate matrix forms independent variables and corresponding date of delivery forms the target variable. The subset size of the rows could be an x percentage of the original size, wherein x has a value >50. Further, at step 314, the prediction errors of the regressor models generated during k-folds cross validation performed on the subset of the multivariate matrix are computed. At step 316, each pregnancy window corresponds to a pregnancy duration is made progressively lesser by one week as compared to the previous or the next pregnancy window. Further the steps of training the regressor model with k-fold cross validation for a particular metagenomic feature is repeated, each time using input data corresponding to one of a plurality of pregnancy windows.

In the next step 318, corresponding prediction errors are computed for input data corresponding to each pregnancy window. At step 320, a predefined number of top pregnancy windows which resulted in the smallest prediction errors is identified for each metagenomic feature. Further at step 322, a pregnancy window that occurs the maximum number of times across all metagenomic features is selected, from amongst identified predefined number of top pregnancy windows. At step 324, the respective regressor model is retrieved for each metagenomic feature, corresponding to the selected pregnancy window the regressor model comprises a plurality of temporal features.

According to an embodiment of the present disclosure the steps of selecting optimum and early temporal window of sample collection can also be explained with the help of following example scenario:

The total duration of pregnancy has been seen to vary between (as low as) 120 or less and (as high as) 280 days or more, it would not be cost and operationally effective to collect too many samples spread across entire duration of pregnancy to predict the day of delivery of a child. Any prediction of expected day of delivery of a child would be of effective utility if it is not only accurate but also predicted early in the pregnancy with as less samples as possible. Selection of important days in pregnancy that can potentially aid prediction of the day of delivery is therefore critical. Following process is adopted in this regard:

1. For each of the multi-variate matrix constructed at step 310, the original day of delivery for each woman is recorded. This serves as the target to be regressed. The days of sample collection serve as the features while the pregnant subjects (participants) for which abundance of the matrix specific microbe were computed in step 1 serve as observations.

2. A subset (e.g., 10%) of observations (i.e., matrices for 10% women) are set aside for held-out testing.

3. Remaining data for 90% women, termed training data, is further divided into sub-train and cross validation set through a 90:10 split of training data (i.e., 10-fold cross validation subsets).

4. Day of delivery is first regressed from the complete sub-training matrix (i.e., without any trimming of any feature) using a machine learning algorithm. In other words, given each matrix represents temporal abundance profile of a given microbe or a diversity metric (or any other metagenomic marker) in different women, one microbe or metric specific model is generated from each matrix that can predict the date of delivery of the unborn child

5. For the model so generated, features (i.e., days of pregnancy) with non-zero importance (or greater than a threshold value) are noted from the model attributed importance or coefficients to the constituent features. Model error (such as root mean square error, RMSE, in days) is also noted in validation set.

6. The sub-train matrix feature space is next reduced by removing one week (or 7-day data) from the chronologically latest feature set (days of pregnancy) in the matrix of step 3.

7. New model is now developed for the microbe specific reduced matrix and important days as well as errors are noted once again

8. The successive reduction in feature space is continued by deleting another 7 days from the previously reduced matrix and creating a new model with stored important days and error of the model. This is continued till the total matrix is reduced to one week i.e., first 7 days of pregnancy.

9. Total 40 models are therefore developed for each microbe or metric (1 model for entire matrix and 39 models for reduced forms of the matrix) in one iteration of creating a random sub-train and validation set. For each model, error and important days are stored.

10. Steps 3-9 are repeated for 9 more folds to create a 10-fold validation.

11. Error profile is plotted in the form of box plots for all 40 models of the microbe specific to each matrix type (untrimmed, reduced/trimmed) and a sorted list of important days is generated for the microbe by aggregating the stored importance across all models across all folds.

12. This process of step 3-11 in mentioned above is repeated for all the matrices (i.e., metagenomic markers) constructed in step 310 of the flowchart 300 resulting in N x 40 x 10 models (where N is number of metagenomic markers i.e., one or more of OTUs or diversity metrics)

13. Low common minima in error profile is observed for all N metagenomic markers in individual box plots for each marker, each of which was subjected to creation of 40 models in each fold, and whose error was plotted.

14. Low common minima refer to the common reduced matrix point against which a low median error is observed. From an ascending ordered list of errors for each window, the top 5 window indices are noted for each feature. The most commonly occurring window across the features is selected.

15. From an ascending ordered list of errors for each window, the top 5 window indices are noted for each feature. The most commonly occurring window across the features is selected.

16. In case of ties or comparable errors across multiple reduced matrices, the more reduced matrix point is preferred. Notably, if different marker specific error profiles have minima in neighborhood i.e., if there is none exact common minima (1-2 week reduction around one another), the larger sized matrix point of the said range will be selected.

17. Keeping the largest day of the low common minima point as reference, important days are fetched from the sorted list of important days by ignoring days that do not fall within the upper limit of selected reduced matrix point. If many of the important days selected are consecutive or have narrow difference in days, then only those days having a minimum gap of 4 days from each other are selected. In alternate implementations, this day difference can be set to a different value other than 4.

18. If the number of important days derived from the selected window after day differencing protocol (detailed in the previous step) comes out to be less than 15, then earlier days that fall prior to the earliest day are recursively added to the important day list which have at least 4-day difference. In alternate implementations, some other day difference such as at weekly or monthly level can be chosen, depending upon the number of days to fill the 15 days collection threshold.

19. If no common minima are observed, 112 is set as the upper limit point and days within this range are selected as important days from the aggregate sorted list, on which samples should be collected in pregnant women. If less than 15 important days are observed, weekly samples starting from second week of pregnancy are added to the important days to ensure that at least 15 samples are collected.

Further at step 326 of the method 300 of building the ensemble, the plurality of temporal features of the regressor model is sorted based on their importance scores and selecting, a set of top scoring temporal features. The selected set of temporal features refer to a specific set of days within the selected pregnancy window. Further at step 336, the steps of retrieving and sorting are repeated for each of the metagenomic features in microbiome profile data. At step 328, a predefined number of days are identified and selected that are associated to the metagenomic features, from amongst the days occurring in the selected pregnancy window. At step 330, a feature-specific regressor model is generated for each metagenomic feature, with a k-fold cross validation on a custom multi-variate matrix comprising of data pertaining to the numeric values of the features on the identified specific subset of days. In the next step 332, a subset of models is selected out of the models trained, wherein the selected models have the lowest prediction error. In another implementations, the prediction error could be further lowered as more samples are gathered from the pregnant subjects. And finally, at step 334, an ensemble is built using the selected subset of models.

According to an embodiment of the present disclosure, it is observed that the ensemble of the regression models which is intelligently created by performing aggregation of two or more regression models from the models trained specific to the one or more composition features, provides an accurate prediction of the delivery date of the pregnant subject. Different combinations of aggregation of regression models are available specific to the stage of pregnancy, age of the pregnant subject, race, geography, possibility of pre-term/term, schedule of sample collection, and history of pregnancy of the pregnant subject.

According to an embodiment of the disclosure, the predictive model is developed on the 90% training data obtained earlier. Given each microbe or metagenomic marker specific temporal abundance matrix can generate a predictive model, an ensembling process is utilized to develop the ensemble as follows:

- For each microbe specific model development, only those days are employed which were selected in the end of step 328. A regressor is developed on 90% of training data using the single microbe specific temporal abundance profile across different pregnant as obtained in step 310 but trimmed to the days selected in step 328. Model prediction is performed on the validation set. This is repeated 5-fold to record error in each fold and obtain mean error across all 5 folds.

- Once all predictors are developed using temporal abundance profiles of individual microbes or markers and their errors & average errors recorded across 5-fold cross validation, an ensemble through regression is started -

Approach 1: Top 10 metagenomic features selected based on a lowest central value tendency of RMSE (root mean square error). Ensemble prediction error is computed by taking the mean of average predictions from top 10 features with lowest lowest central value tendency RMSE.

Approach 2: Detailed below:

- First a least error model based on cross-validation average error is selected. Multiple models are selected if they share same or similar error (i.e. less than 2-day error).
- Then all models with individual average predictions across all cross folds within 2-10 days less than the date predicted by the least error model are selected. The average prediction of these models in same cross fold (e.g. average of their individual predictions in first cross fold) is separately retained for each fold. These are termed as early prediction model set.
- Then all models with average predictions within 2-10 days more than date predicted by the least error model are selected. The average prediction of these models in same cross fold (e.g. average of their individual predictions in first cross fold) is separately retained for each fold. These are termed as late prediction model set.
- Average prediction of all these models serves as the predicted day of delivery which is computed for all 5 folds previously performed for each of these individual models. Errors are computed on this average prediction.

In alternate implementation, predicted day of delivery can be indicated as an approximation which may lie anywhere between average of early and late prediction model set. In alternate implementation, the average between those set of models (among least error set, early prediction set, and late prediction set) is performed whose average predictions are closer to other set's average prediction.

In alternate implementation, the dataset used in method 200 can be composed of both preterm and term specific microbiome samples from the plurality of pregnant subjects, and the subsequent ensemble generated from the method 200 may be used to predict the EDD. The same ensemble can therefore be used as a classifier for term and preterm prediction for women with unknown EDD, wherein the classifier indicates preterm condition when the EDD using the ensemble is lower than 259th day of pregnancy or 37th week and term condition otherwise.

According to an embodiment of the disclosure, machine learning algorithm can be one or more of a decision tree-based regressor, a boosted regressor, a linear regressor, a logistic regressor or any other regression-based machine learning method. In alternate implementations, pre-term or term delivery outcome specific models can be built and these can be utilized for estimating day of delivery by first predicting the nature of delivery using existing pre-term delivery predictors. Further, different validation strategies may be adopted for temporal and metagenomic features.

Each ensembling of models can be wrapped in a reinforcement learning framework, such that anonymized data from the volunteering pregnant subject, who wish to disclose the outcome of the delivery and contribute the data to the emergency management system of the hospital, is added to the system and dynamic improvement in the parameters of all the model aggregates is performed by estimating a quantum of error of predictions made for the contributing pregnant subject, against the original outcome. This enables up-gradation of all sets of models used in the clinical setting by re-executing the model development cycle.

According to the present disclosure, the method further comprises deriving/applying a therapeutic solution to improve the chances of term delivery. The therapeutic solutions can be applied in at least one of the following forms: 1) As topical ointment in form of paste/ liquid/ gel/ powder 2) As a spray, roller or other forms of application 3) As an injection 4) Probiotic and prebiotic supplements (microbes/ microbial products/ chemicals) can be in form of drinks, tablets and which boost the population of the intended microbes or suppress unintended microbes 4) Any other mode of drug delivery other than above mentioned which would be applicable to the pregnant subject predicted to have pre-term delivery are within the scope of this application.

According to an embodiment of the present disclosure, the system 100 also configured to use the expected delivery date for managing the healthcare emergency services needed for maternal and neonatal healthcare, wherein in one exemplary embodiment, the delivery date of the pregnant subject is predicted by application of at least one of the microbiome, or microbiome-associated properties e.g. bacteria, bacterial diversity, bacterial proteins, bacterial metabolites, bacterial metabolic pathways, in conjugation with biological features of a pregnant subject and features of an unborn child/foetus (e.g. age, blood biochemistry).

The system 100 further configured to compute 'weekly predicted emergency burden (wPEB)' metrics associated with various units/entities which are critical to handle the healthcare emergency, based on the registered pregnant patient's information submitted to the hospital. Further, the disclosed method comprises generating an alert as soon as the weekly predicted emergency burden (wPEB)' metrics associated with any of various units/entities which are critical to handle the healthcare emergency goes below 1, then the system will create an alert to prepare in advance for adverse emergency requirement/events related to maternity or neonatal requirement at the hospital.

According to an embodiment of the disclosure, the disclosure provides a solution that provides (i) creation of a digital 'logistic and communicative' machine deployable framework that enable before time planning of maternal and neonatal healthcare emergency management, including hospital resource management ahead of time and performing planning of maternal and neonatal healthcare emergency management based on the EDD of the pregnant subject, (ii) In order to predict the delivery date of the pregnant subject, identifying microbiome-based compositions and microbiome based signatures pertaining to the pregnant subjects that enables development of an accurate model for predicting the expected date of birth of an unborn child/ a delivery date of the pregnant subject using microbiome sample(s) and other biological/ physiological or metabolic features obtained from the pregnant subject, (iii) an accurate predictive model for estimating a day/ date of delivery of pregnant subject / an unborn child using microbiome sample(s) and biological features obtained from a pregnant woman, (iv) creation of digital machine deployable model for mobile applications, web based applications and other software services needed for automation of the above mentioned predictive model and associate system for execution of the predictive model The suggested microbial compositions can be used for an effective probiotic supplement needed for correcting the population of microbes in a pregnant woman for managing the day of delivery of the pregnant subject.

According to an embodiment of the disclosure, the system 100 is also configured to the one or more entities which are critical/key role player in handling the medical emergency in the maternity hospital and/or neonatal care units comprises Hospital beds, medicines, doctors, Nurse staff, and the like. The weekly predicted emergency burden for each of the entities comprising Bed wPEB, Medicine wPEB (weekly Predicted Emergency Burden), Doctor wPEB and nursing staff wPEB. The wPEB (weekly Predicted Emergency Burden) for each of the above- mentioned entities is computed using the predicted delivery date of the pregnant subjects and information registered for the pregnant subjects. The wPEB (weekly Predicted Emergency Burden) for each entity is computed using the below mentioned formula:

$$\text{weekly Predicted Emergency Burden (wPEB)} = 7 * (ART + ARP) / [(TD * MR) + (PD * MR)]$$

wherein, T indicates Term Delivery from 'ART', P indicates Preterm delivery from ARP, AR from ART or ARP refers to daily availability of units of required index specific to the type of delivery (e.g. bed, doctors, staff, medicine etc) including T (term delivery) or P (preterm delivery) therefore ART is daily Available Resources/units for Term delivery and ARP is daily available resources/units for preterm delivery, TD refers to total term deliveries expected within the given week, PD refers to total pre-term deliveries expected within the given week, MR refers to mean weekly requirement in the hospital for the term deliveries in the last one year.

The system 100 is also configured to automatically generate an alert to prepare in advance for adverse emergency requirement/events related to maternity or neonatal requirement at the hospital when value of wPEB is lower than 1 for any type of wPEB comprising Bed wPEB, Medicine wPEB, Doctor wPEB and nursing staff wPEB. Thus, generating an alert for preparing in advance for adverse emergency requirement/events related to maternity or neonatal requirement at the hospital when value of any of wPEB as mentioned above is less than 1 enables in advance planning and management of handling the emergency related to maternal or neonatal healthcare emergency which may happen as the alert is specifically generated based on the EDD of the pregnant subjects which are registered with the hospital or healthcare service.

The system 100 is also configured to update a database of the system 100 available in the IoT framework with one or more of 'weekly predicted emergency burden (wPEB)' metrics based on the registered pregnant patient's information submitted in the hospital. The computations of wPEB are passed from the analyser using the system Application Program Interface (API) over the internet to a database of above mentioned IoT based platform hosted in hospital servers. The System API updates the database with one or more of 'weekly predicted emergency burden (wPEB)' metrics (as mentioned below) based on the registered pregnant patients' information submitted in the hospital. The system API also updates the database with total estimated deliveries predicted within a given week from the registered pregnant patients' information in the hospital. In one or more examples, the wPEBs may refer to: Bed wPEB, Medicine wPEB, Doctor wPEB and nursing staff wPEB and the like.

A case transfer recommendation system is additionally available in the presently disclosed systems for one or more networks of hospitals sharing a code of communication or logistic sharing in the event of adverse emergency requirement/events. The case transfer recommendation system enables accessing any of medically relevant hospital resources or medical equipment or healthcare resources using the presently disclose system installed over IoT based framework. In present aspect, limitations to beds, staff, medicines and doctors not to be assumed.

**Experimental data**

According to an embodiment of the disclosure, the system 100 can also be explained with the help of experimental data. For this purpose, vaginal microbiome data is first taken from a research publication: "Fettweis, J.M., Serrano, M.G., Brooks, J.P. et al - The vaginal microbiome and preterm birth. Nat Med 25, 1012-1021 (2019)", that comprised of 134 pregnant subjects. This number was reduced to 120 by filtering out those subjects which had less than 6 collected samples and filtering those for whom the racial/ethnicity information was missing. Two different models were developed and trained by splitting the vaginal microbiome data of 120 pregnant women into 39 preterm and 81 term specific data segments. In an implementation, 5 preterm women samples and 10 term women samples were selected randomly using the 'random' package with seed=1000 in python to create held-out test sets for preterm and term specific model training.

According to an embodiment of the disclosure, different algorithms for getting values for missing days are taken to be K-nearest neighbour (KNN) for data imputation, AdaBoost decision tree as a regressor, a time-series interpolator, and ARIMA for autoregressive moving averaging. For each pre-filtered feature for any subject, the missing day's values were computed as follows:

a) First, three different models are created using the available feature data for the collected days for a subject. These models are: AdaBoost regressor, KNN imputer and time-series linear interpolator. All these models use collection day as the independent variable and feature abundance/value as the dependent variable. The linear interpolar model only predicts days in between the earliest and latest collected dates. The KNN model imputes the missing data using the entire available feature space that were originally present for a host prior to pre-filtering.

b) Secondly, the mean of the predicted values for the uncollected days is taken from each of the three models for a given feature. The mean forms the baseline "flat and evenly spaced" trend for a feature across the duration of the pregnancy. However, unvarying repetition of values is observed, particularly for those dates having large gaps between collection.

c) Lastly, an autoregressive ARIMA model is made using the above baseline trend for all the days, collected and uncollected. A walk-forward forecasting strategy is adopted, in which at each step the last 'n order' auto-regressive days are used to forecast the next day's value, which is then again used for the next iteration. This strategy is applied separately in three levels:

i) First within the date range starting from the earliest collected sample to the latest.
ii) Second, from the days beyond the last collected date to Day 280.
iii) Finally, A back-propagation strategy which reverses the trend and predicts from the earliest collected date all the way to Day 1.

The specifications of the different types of models are listed below:

a) KNN Imputer: Performed using the KNN impute module of the 'sklearn' package in python. The number of neighbouring samples is set to '3', which are given equal weightage by setting the weights parameter to 'uniform'. The rest of the parameters are set to default.
b) AdaBoost Regressor: Performed using the 'AdaBoost Regressor' ensemble module of the 'sklearn' package in python. Here, the number of estimators were taken as 50, while keeping other parameters as default.
c) Time-series interpolator: Performed using the 'TimeSeries' class of the 'traces' python package. All parameters were set to default.
d) ARIMA: Autoregressive integrated moving average model was developed using the "statsmodels' package in python. The number of autoregressive terms were set to 10, the differencing was set to 1 and the number of lagged forecast errors were set to 0.

According to another embodiment of the present disclosure, a preprocessing filtering method can also be employed to reduce the metagenomic feature space from over a thousand to few hundreds. This initial set of metagenomic features were filtered based on prevalence and relevance of these features across the 120 subjects. Relevance criteria indicated that a taxon had at least $10^{-4}$ relative abundance value in at least 75% of the collected samples for a particular subject. Then, the counts of these metagenomic features across all hosts were determined and only those were selected which were present in at least 25 subjects (i.e., in approximately 25% of the total subjects), thus defining the feature prevalence.

Within this implementation, if any of the metagenomic feature from the prefiltered list is absent within a host, either due to simple non-existence or by extremely low relative abundance in 75% collected days, then these features are not ignored for model training, but rather their values across entire pregnancy period are treated as zero.

In an example, the number of features left were 189 after metagenomic feature space reduction treatment. On including the six diversity indices, the total came to 195 microbiome relevant features. In another implementations, a different threshold value of relevance criterion, such as $10^{-6}$ relative abundance value, and a different threshold value for prevalence criterion, such as > 5% of total subjects examined, can be adopted to widen the feature space for a more generalized implementation of taxonomic feature selection. In yet another implementations, feature space reduction can be limited to race, ethnicity or term/preterm specific subject groups for the purpose of training the models detailed in the subsequent steps of the present disclosure.

In an implementation, from the selected low common minima window, top 30 most common important days among the chosen features were selected for both preterm and term datasets (Refer TABLE 1). In alternate implementations, the number of important days from the common minima window could be changed to other numerical value.

In an implementation, random forest regressor (RFR) model was trained on the remaining training data subsets for the term and preterm delivery conditions separately. Each RFR model was built using sklearn package, where number of estimators were kept as 100, random state of the model was set to 999, and other parameters were set to default. Each feature-specific multi-variate dataset has its own RFR model and thus, each fold of the cross-validation step produced error value for every cross-validation fold. A threshold value of 0.005 was set for selecting important days for each fold of RFR model pertaining to a given feature dataset.

TABLE 1: From the selected low common minima window, top most common important days among the metagenomic features at different steps were selected for preterm and term datasets

|  | Preterm | Term |
|---|---|---|
| **Data Specific Feature counts (out of 195)** | 71 | 142 |
| **Chosen Window** | 132 | 153 |
| **Occurrence Counts of Chosen Window** | 18 | 39 |
| **Important Days List from chosen window (top 30)** | [98, 104, 105, 106, 107, 108, 109, 110, 111, 112, 113, 114, 115, 116, 117, 118, 119, 120, 121, 122, 123, 124, 125, 126, 127, 128, 129, 130, 131, 132] | [112, 113, 114, 115, 116, 117, 118, 119, 120, 121, 122, 123, 127, 128, 130, 132, 135, 137, 139, 140, 141, 144, 145, 146, 148, 149, 150, 151, 152, 153] |
| **Important Days List after 4-day differencing** | [98, 104, 108, 112, 116, 120, 124, 128] | [112, 116, 120, 127, 132, 137, 141, 145, 149] |
| **Important Days after adding prior days to get minimum 15 days** | [70, 74, 78, 82, 86, 90, 94, 98, 104, 108, 112, 116, 120, 124, 128] | [88, 92, 96, 100, 104, 108, 112, 116, 120, 127, 132, 137, 141, 145, 149] |

In the previous exemplary implementation, the ensemble of 10 feature models related to term data yielded an average prediction error of 3.7 days on the held-out set. Similarly, the RMSE for this term ensemble came out to 4.4 days. For the preterm specific models, the ensemble prediction error was 14.6 days on preterm held-out set while the RMSE of the same was 15.7 days. The selected features forming the ensemble for both datasets are listed in TABLE 2 along with their prediction errors in training and testing stages. The corresponding sequences of OTU specific features are listed in TABLE 3.

| **Ensemble Features (Term)** | **Training Data Median Error (RMSE in days)** | **Held-out Set Average Error (RMSE in days)** |
|---|---|---|
| Prevotellaceae | 5.71 | 5.43 |
| shannon | 6.29 | 4.52 |
| OTU_32 | 6.38 | 5.61 |
| Micrococcaceae | 6.62 | 8.2 |
| Finegoldia | 6.66 | 5.32 |
| Megasphaera | 6.67 | 4.76 |
| OTU_109 | 6.70 | 5.35 |
| peven | 6.80 | 4.19 |
| Prevotella | 6.83 | 5.54 |
| OTU_113 | 6.86 | 5.9 |
|  |  |  |
| **Ensemble Features (Preterm)** | **Training Data Median Error** | **Held-out Set Average Error** |
| OTU_24 | 13.385 | 20.13 |
| Porphyromonadaceae | 15.720 | 20.04 |
| OTU_55 | 16.430 | 21.34 |
| Porphyromonas | 16.455 | 9.82 |
| Aerococcaceae | 16.470 | 15.05 |
| Prevotellaceae | 16.910 | 21.73 |

(continued)

| Ensemble Features (Preterm) | Training Data Median Error | Held-out Set Average Error |
|---|---|---|
| Bifidobacterium | 17.570 | 18.06 |
| Gardnerella | 17.645 | 24.73 |
| Fusobacterium | 17.920 | 12.74 |
| OTU_193 | 17.990 | 15.34 |
| TABLE 2: Features and their individual prediction errors for the ensembles related to term and preterm datasets | | |

TABLE 3: Sequences of the OTU specific features found in the ensembles of preterm and term specific datasets

| Features/ OTU No | OTU Size | Sequence Listing |
|---|---|---|
| OTU_32 | 1197788 | TACGGAAGGTCCGGGCGTTATCCGGATTTATTGGGTTTAAAGGGAG CGTAGGCCGCGTTTTAAGCGTGTTGTGAAATGTAGGCGCCCAACGT CTGCATCGCAGCGCGAACTGGAACGCTTGAGTACGCGCAACGTTG GCGGAATTCGTCGTGTAGCGGTGAAATGCTTAGATATGACGAAGA ACTCCGATTGCGAAGGCAGCTGACGGGAGCGGCACTGACGCTTAA GCTCGAAGGTGCGGGTATCAAACAGG |
| OTU_109 | 242872 | TACGTATGGAGCGAGCGTTGTCCGGAATTATTGGGCGTAAAGGGTG CGCAGGCGGCTTTACAAGTTGGATGTGAAATATTGTGGCTCAACCA CAAACGTGCATCCAAAACTGCAAAGCTTGAGTTAAGGAGAGGTAA GTGGAATTCCTGGTGTAGCGGTGGAATGCGTAGATATCAGGAGGA ATACCGGTGGCGAAGGCGACTTACTGGACTTAAACTGACGCTCAG GCACGAAAGCGTGGGGAGCAAACAGG |
| OTU_113 | 390073 | TACGTAGGTGGCAAGCGTTATCCGGATTTATTGGGCGTAAAGCGAG CGCAGGCGGTTGTTTAGGTCTGATGTGAAAGCCTTCGGCTTAACCG AAGAAGTGCATCGGAAACCGGGCGACTTGAGTGCAGAAGAGGACA GTGGAACTCCATGTGTAGCGGTGGAATGCGTAGATATATGGAAGA |
| | | ACACCAGTGGCGAAGGCGGCTGTCTGGTCTGCAACTGACGCTGAG GCTCGAAAGCATGGGTAGCGAACAGG |
| OTU_24 | 1495517 | TACGTAGGGTGCGAGCGTTGTCCGGAATTACTGGGCGTAAAGGGCT CGTAGGTGGTTTGTCGCGTCGTCTGTGAAATTCCGGGGCTTAACTC CGGGCGTGCAGGCGATACGGGCATAACTTGAGTACTGTAGGGGTA ACTGGAATTCCTGGTGTAGCGGTGAAATGCGCAGATATCAGGAGG AACACCGATGGCGAAGGCAGGTTACTGGGCAGTTACTGACGCTGA GGAGCGAAAGCATGGGTAGCGAACAGG |

(continued)

| Features/ OTU No | OTU Size | Sequence Listing |
|---|---|---|
| OTU_55 | 744012 | TACGGAAGGTCCGGGCGTTATCCGGATTTATTGGGTTTAAAGGGAG CGTAGGCCGCCAGATAAGCGTGTTGTGAAATGTACCGGCTCAACCG GTGAATTGCAGCGCGAACTGTTTGGCTTGAGTGCACGGTAAGCAGG CGGAATTCATGGTGTAGCGGTGAAATGCTTAGATATCATGAAGAAC TCCGATTGCGAAGGCAGCTTGCTGCAGTGCGACTGACGCTGATGCT CGAAGGTGCGGGTATCAAACAGG |
| OTU_193 | 299521 | TACGTAGGTGGCAAGCGTTGTCCGGAATTATTGGGCGTAAAGCGCG CGCAGGCGGCTTACTAAGTCCATCTTAAAAGTGCGGGGCTTAACCC CGTGATGGGATGGAAACTGGAAAGCTGGAGTATCGGAGAGGAAAG TGGAATTCCTAGTGTAGCGGTGAAATGCGTAGAGATTAGGAAGAA CACCGGTGGCGAAGGCGACTTTCTGGACGAAAACTGACGCTGAGG CGCGAAAGCGTGGGGAGCAAACAGG |

A microbial pathway abundance profile can also be obtained by assessing the annotated genomes of all the strains corresponding to microbial genera already identified and the same can also be used in conjugation with a plurality of microbial features. Any other microbial or host feature (morphological, biochemical, metabolic, physical etc.) can also be employed in the regression model development and clinical testing process as described above.

Use of any other size of training and test sample sets is within the scope of the invention. Use of different data sources, larger or smaller than the presented data of 134 pregnant women is also within the scope of this invention.

In another implementation, a mathematical model is developed for analogous computation of predicted day of delivery of the pregnant subject in absence of electronic/communicative/internet facilities, such that given the identification of abundance of different microbial features, a mathematical formula can be employed to arrive at an approximate day of delivery.

According to an embodiment of the disclosure, the disclosure provides a solution that involves in creating a plurality of microbial features, referred to as microbiome data or as a microbiome profile, that must include, but not limited to microbial genus-level abundance profile, microbial family-level abundance profile, microbial sequence cluster profile and microbial diversity profile using a biological sample (e.g. vaginal swab, saliva, stool etc) from a pregnant woman, where microbial genus and family level abundance profile is created by processing the 16S rDNA or WGS sequence data specific to microbes using EBI Mgnify (or any other tool or classifier like RDP, GreenGenes, SILVA, Mothur etc is well within the scope of this invention); microbial sequence cluster file refers to a file that contains sets of de-novo learnt clusters of similar sequences using global pairwise sequence comparison between microbial 16S sequences extracted from the sample taken from a pregnant woman. Creating a normalized genus abundance table, where normalization is done by dividing the number of sequences assigned to each genus by the median 16S gene copy number found in genomes of all the strains corresponding to said genus. Conjugating the normalized genus table with the plurality of microbial features. Creating an alpha diversity table using the sequence cluster file, the table specifically containing the number of unique sequences, Simpson-evenness index and Shannon index. Conjugating the alpha diversity table with the plurality of microbial features. Conjugating biological features of the same as well as other samples from the same woman, other than the microbial features (e.g. biochemistry, age etc), with the plurality of microbial features to create a global feature table.

The prediction outcome is generated in a digital system consisting of an internet-enabled qPCR machine or a DNA sequencing machine or any machine that can assess microbial features in the microbiome sample. The assessments are passed through LAN/WIFI/BLUETOOTH/INFRARED or (MANUALLY in case of internet or power outages or communication bottlenecks) or other data transmission methods to an analyzer hardware with prediction software installed containing models in the form of CML files (other formats of model files are well within the scope of this invention). The analyzer hardware is enabled by a digital screen as well as an analog meter to display prediction for the date of birth / the day of delivery of the pregnant subject. The use of mobile devices, vehicle embedded machines, portable machines, analogue analysis equipment for implementation of the system for prediction of day of delivery of the pregnant subject is well within the scope of this invention.

The written description describes the subject matter herein to enable any person skilled in the art to make and use the embodiments. The scope of the subject matter embodiments is defined by the claims and may include other modifications that occur to those skilled in the art. Such other modifications are intended to be within the scope of the claims if they have similar elements that do not differ from the literal language of the claims or if they include equivalent elements with insubstantial differences from the literal language of the claims.

The embodiments of present disclosure herein address unresolved problems related to accurate and timely estimation of EDD in the pregnant subject. Further the disclosure also addresses the challenges associated with the management of delivery of the child. The embodiment thus provides a method and system for estimation of an expected delivery date (EDD) of a pregnant subject using microbiome data

It is to be understood that the scope of the protection is extended to such a program and in addition to a computer-readable means having a message therein; such computer-readable storage means contain program-code means for implementation of one or more steps of the method, when the program runs on a server or mobile device or any suitable programmable device. The hardware device can be any kind of device which can be programmed including e.g. any kind of computer like a server or a personal computer, or the like, or any combination thereof. The device may also include means which could be e.g. hardware means like e.g. an application-specific integrated circuit (ASIC), a field-programmable gate array (FPGA), or a combination of hardware and software means, e.g. an ASIC and an FPGA, or at least one microprocessor and at least one memory with software processing components located therein. Thus, the means can include both hardware means and software means. The method embodiments described herein could be implemented in hardware and software. The device may also include software means. Alternatively, the embodiments may be implemented on different hardware devices, e.g. using a plurality of CPUs.

The embodiments herein can comprise hardware and software elements. The embodiments that are implemented in software include but are not limited to, firmware, resident software, microcode, etc. The functions performed by various components described herein may be implemented in other components or combinations of other components. For the purposes of this description, a computer-usable or computer readable medium can be any apparatus that can comprise, store, communicate, propagate, or transport the program for use by or in connection with the instruction execution system, apparatus, or device.

The illustrated steps are set out to explain the exemplary embodiments shown, and it should be anticipated that ongoing technological development will change the manner in which particular functions are performed. These examples are presented herein for purposes of illustration, and not limitation. Further, the boundaries of the functional building blocks have been arbitrarily defined herein for the convenience of the description. Alternative boundaries can be defined so long as the specified functions and relationships thereof are appropriately performed. Alternatives (including equivalents, extensions, variations, deviations, etc., of those described herein) will be apparent to persons skilled in the relevant art(s) based on the teachings contained herein. Such alternatives fall within the scope of the disclosed embodiments. Also, the words "comprising," "having," "containing," and "including," and other similar forms are intended to be equivalent in meaning and be open ended in that an item or items following any one of these words is not meant to be an exhaustive listing of such item or items, or meant to be limited to only the listed item or items. It must also be noted that as used herein and in the appended claims, the singular forms "a," "an," and "the" include plural references unless the context clearly dictates otherwise.

Furthermore, one or more computer-readable storage media may be utilized in implementing embodiments consistent with the present disclosure. A computer-readable storage medium refers to any type of physical memory on which information or data readable by a processor may be stored. Thus, a computer-readable storage medium may store instructions for execution by one or more processors, including instructions for causing the processor(s) to perform steps or stages consistent with the embodiments described herein. The term "computer-readable medium" should be understood to include tangible items and exclude carrier waves and transient signals, i.e., be non-transitory. Examples include random access memory (RAM), read-only memory (ROM), volatile memory, nonvolatile memory, hard drives, CD ROMs, DVDs, flash drives, disks, and any other known physical storage media.

**Claims**

1. A processor implemented method (200) for estimation of an expected delivery date (EDD) of a pregnant subject using microbiome data, the method comprising:

   collecting a set of biological samples on a predefined set of days from the pregnant subject whose EDD is to be estimated (202);
   performing, via one or more hardware processors, microbiome profiling for each of the collected biological samples to obtain a microbiome profile data comprising of a plurality of metagenomic features (204);
   predicting, via the one or more hardware processors, a set of expected delivery dates for the pregnant subject by providing the microbiome profile data as input to a set of models present in a prebuilt ensemble (206); and calculating, via the one or more hardware processors, a central tendency value of the predicted set of expected delivery dates to determine the EDD of the pregnant subject, wherein the predefined set of days are pre-ascertained, and the ensemble is built using the steps of (208):

collecting biological samples from a plurality of pregnant subjects at predefined time points during a period of pregnancy (302);

performing microbiome profiling for each of the collected biological samples to obtain a microbiome profile data comprising of the plurality of metagenomic features (304);

training one or a combination of regressors using the obtained microbiome profile data (306);

reconstructing, using the trained regressors, for each pregnant subject among the plurality of pregnant subjects, a simulated microbiome profile, wherein the simulated microbiome profile comprises of values in original microbiome profile data and reconstructed values of all metagenomic features for specific days of the pregnancy duration when biological samples were not collected from the pregnant subject (308);

constructing, using the simulated microbiome profile, a multi-variate matrix for each of the metagenomic features, wherein the multi-variate matrix refers to the microbiome profile of the metagenomic feature observed in each of the pregnant subjects across entire duration of pregnancy, wherein each matrix comprises a unique participant ID corresponding to each pregnant subject in rows and each day of pregnancy, in which the microbiome profile of the metagenomic feature is observed, as columns (310);

training a regressor model with a k-fold cross validation on a predefined subset of rows in the multi-variate matrix for each metagenomic feature, wherein values of the metagenomic feature across the entire pregnancy duration belonging to the subset of the multi-variate matrix forms independent variables and corresponding date of delivery forms the target variable (312);

computing prediction errors of the regressor models generated during k-folds cross validation performed on the subset of the multi-variate matrix (314);

repeating the step of training the regressor model with k-fold cross validation for a particular metagenomic feature, each time using input data corresponding to one of a plurality of pregnancy windows, wherein each pregnancy window corresponds to a pregnancy duration that is progressively lesser by one week as compared to the previous or the next pregnancy window (316);

computing corresponding prediction errors for input data corresponding to each pregnancy window (318);

identifying, for each metagenomic feature, a predefined number of top pregnancy windows which resulted in the smallest prediction errors (320);

selecting, from amongst identified predefined number of top pregnancy windows, a pregnancy window that occurs the maximum number of times across all metagenomic features (322);

retrieving, for each metagenomic feature, the respective regressor model corresponding to the selected pregnancy window the regressor model comprises a plurality of temporal features (324);

sorting the plurality of temporal features of the regressor model based on their importance scores and selecting, a set of top scoring temporal features, wherein the selected set of temporal features refer to a specific set of days within the selected pregnancy window (326);

repeating the steps of retrieving and sorting for each of the metagenomic features in microbiome profile data (336);

identifying and selecting, from amongst the specific set of days occurring in the selected pregnancy window, a predefined number of days that are associated to the metagenomic features (328);

generating, for each metagenomic feature, a feature-specific regressor model with a k-fold cross validation on a custom multi-variate matrix comprising of data pertaining to the numeric values of the features on the identified specific subset of days (330);

selecting a subset of models out of the models trained, wherein the selected models have the lowest prediction error (332); and

forming an ensemble using the selected subset of models (334).

2. The processor implemented method of claim 1 comprising planning and managing healthcare emergency services in a healthcare unit using the expected delivery date of pregnant subject.

3. The processor implemented method of claim 1 comprising computing 'weekly predicted emergency burden (wPEB)' metrics associated with the healthcare unit which are critical to handle the healthcare emergency, based on the registered pregnant subject's information submitted to the healthcare unit.

4. The processor implemented method of claim 3, wherein the wPEB metrices comprises Bed wPEB, Medicine wPEB, Doctor wPEB and nursing staff wPEB.

5. The processor implemented method of claim 1, wherein the central tendency value is one of a mean or a median of the predicted set of expected delivery dates.

6. The processor implemented method of claim 1, wherein the microbiome samples refer to one of stool sample, tissue samples from body sites, a swab, and saliva.

7. The processor implemented method of claim 1, wherein the plurality of metagenomic features comprises one or both of microbial features and host features.

8. The processor implemented method of claim 1, wherein Day 1 and Day 280 of the pregnancy duration serve as the boundaries of obtaining microbiome characterization data.

9. The processor implemented method of claim 1, wherein the plurality of microbial and biological features comprises a microbial genus-level abundance profile, a microbial family-level abundance profile, a microbial sequence cluster profile, a microbial diversity profile and a normalized genus abundance table.

10. The processor implemented method of claim 1, wherein the subset size of the rows is more than 50 percent of the original size.

11. The processor implemented method of claim 1, wherein the microbiome profile data also obtained from a plurality of non-microbiome sources comprising physiological data, biochemical data, and host-specific data corresponding to the pregnant subject.

12. A system (100) for estimation of an expected delivery date (EDD) of a pregnant subject using microbiome data, the system comprises:

> a microbiome sample receiver (114) for collecting a set of biological samples on a predefined set of days from the pregnant subject whose EDD is to be estimated;
> a microbiome characterization platform (116) for performing microbiome profiling for each of the collected biological samples to obtain a microbiome profile data comprising of a plurality of metagenomic features;
> one or more hardware processors (110); and
> a memory (108) in communication with the one or more hardware processors, wherein the one or more first hardware processors are configured to execute programmed instructions stored in the one or more first memories, to:
>
>> predict a set of expected delivery dates for the pregnant subject by providing the microbiome profile data as input to a set of models present in a prebuilt ensemble; and
>> calculate a central tendency value of the predicted set of expected delivery dates to determine the EDD of the pregnant subject, wherein the predefined set of days are pre-ascertained, and the ensemble is built using the steps of:
>>
>>> collecting biological samples from a plurality of pregnant subjects at predefined time points during a period of pregnancy;
>>> performing microbiome profiling for each of the collected biological samples to obtain a microbiome profile data comprising of the plurality of metagenomic features;
>>> training one or a combination of regressors using the obtained microbiome profile data;
>>> reconstructing, using the trained regressors, for each pregnant subject among the plurality of pregnant subjects, a simulated microbiome profile, wherein the simulated microbiome profile comprises of values in original microbiome profile data and reconstructed values of all metagenomic features for specific days of the pregnancy duration when biological samples were not collected from the pregnant subject;
>>> constructing, using the simulated microbiome profile, a multi-variate matrix for each of the metagenomic features, wherein the multi-variate matrix refers to the microbiome profile of the metagenomic feature observed in each of the pregnant subjects across entire duration of pregnancy, wherein each matrix comprises a unique participant ID corresponding to each pregnant subject in rows and each day of pregnancy, in which the microbiome profile of the metagenomic feature is observed, as columns;
>>> training a regressor model with a k-fold cross validation on a predefined subset of rows in the multi-variate matrix for each metagenomic feature, wherein values of the metagenomic feature across the entire pregnancy duration belonging to the subset of the multi-variate matrix forms independent variables and corresponding date of delivery forms the target variable;

computing prediction errors of the regressor models generated during k-folds cross validation performed on the subset of the multi-variate matrix;

repeating the step of training the regressor model with k-fold cross validation for a particular metagenomic feature, each time using input data corresponding to one of a plurality of pregnancy windows, wherein each pregnancy window corresponds to a pregnancy duration that is progressively lesser by one week as compared to the previous or the next pregnancy window;

computing corresponding prediction errors for input data corresponding to each pregnancy window;

identifying, for each metagenomic feature, a predefined number of top pregnancy windows which resulted in the smallest prediction errors;

selecting, from amongst identified predefined number of top pregnancy windows, a pregnancy window that occurs the maximum number of times across all metagenomic features;

retrieving, for each metagenomic feature, the respective regressor model corresponding to the selected pregnancy window the regressor model comprises a plurality of temporal features;

sorting the plurality of temporal features of the regressor model based on their importance scores and selecting, a set of top scoring temporal features, wherein the selected set of temporal features refer to a specific set of days within the selected pregnancy window;

repeating the steps of retrieving and sorting for each of the metagenomic features in microbiome profile data;

identifying and selecting, from amongst the specific set of days occurring in the selected pregnancy window, a predefined number of days that are associated to the metagenomic features;

generating, for each metagenomic feature, a feature-specific regressor model with a k-fold cross validation on a custom multivariate matrix comprising of data pertaining to the numeric values of the features on the identified specific subset of days;

selecting a subset of models out of the models trained, wherein the selected models have the lowest prediction error; and

forming an ensemble using the selected subset of models.

13. One or more non-transitory machine-readable information storage mediums comprising one or more instructions which when executed by one or more hardware processors cause:

collecting a set of biological samples on a predefined set of days from the pregnant subject whose EDD is to be estimated;

performing, via one or more hardware processors, microbiome profiling for each of the collected biological samples to obtain a microbiome profile data comprising of a plurality of metagenomic features;

predicting, via the one or more hardware processors, a set of expected delivery dates for the pregnant subject by providing the microbiome profile data as input to a set of models present in a prebuilt ensemble; and

calculating, via the one or more hardware processors, a central tendency value of the predicted set of expected delivery dates to determine the EDD of the pregnant subject, wherein the predefined set of days are pre-ascertained, and the ensemble is built using the steps of:

collecting biological samples from a plurality of pregnant subjects at predefined time points during a period of pregnancy;

performing microbiome profiling for each of the collected biological samples to obtain a microbiome profile data comprising of the plurality of metagenomic features;

training one or a combination of regressors using the obtained microbiome profile data;

reconstructing, using the trained regressors, for each pregnant subject among the plurality of pregnant subjects, a simulated microbiome profile, wherein the simulated microbiome profile comprises of values in original microbiome profile data and reconstructed values of all metagenomic features for specific days of the pregnancy duration when biological samples were not collected from the pregnant subject;

constructing, using the simulated microbiome profile, a multi-variate matrix for each of the metagenomic features, wherein the multi-variate matrix refers to the microbiome profile of the metagenomic feature observed in each of the pregnant subjects across entire duration of pregnancy, wherein each matrix comprises a unique participant ID corresponding to each pregnant subject in rows and each day of pregnancy, in which the microbiome profile of the metagenomic feature is observed, as columns;

training a regressor model with a k-fold cross validation on a predefined subset of rows in the multi-variate matrix for each metagenomic feature, wherein values of the metagenomic feature across the entire pregnancy duration belonging to the subset of the multi-variate matrix forms independent variables and corresponding date of delivery forms the target variable;

computing prediction errors of the regressor models generated during k-folds cross validation performed on the subset of the multi-variate matrix;

repeating the step of training the regressor model with k-fold cross validation for a particular metagenomic feature, each time using input data corresponding to one of a plurality of pregnancy windows, wherein each pregnancy window corresponds to a pregnancy duration that is progressively lesser by one week as compared to the previous or the next pregnancy window;

computing corresponding prediction errors for input data corresponding to each pregnancy window;

identifying, for each metagenomic feature, a predefined number of top pregnancy windows which resulted in the smallest prediction errors;

selecting, from amongst identified predefined number of top pregnancy windows, a pregnancy window that occurs the maximum number of times across all metagenomic features;

retrieving, for each metagenomic feature, the respective regressor model corresponding to the selected pregnancy window the regressor model comprises a plurality of temporal features;

sorting the plurality of temporal features of the regressor model based on their importance scores and selecting, a set of top scoring temporal features, wherein the selected set of temporal features refer to a specific set of days within the selected pregnancy window;

repeating the steps of retrieving and sorting for each of the metagenomic features in microbiome profile data;

identifying and selecting, from amongst the specific set of days occurring in the selected pregnancy window, a predefined number of days that are associated to the metagenomic features;

generating, for each metagenomic feature, a feature-specific regressor model with a k-fold cross validation on a custom multi-variate matrix comprising of data pertaining to the numeric values of the features on the identified specific subset of days;

selecting a subset of models out of the models trained, wherein the selected models have the lowest prediction error; and

forming an ensemble using the selected subset of models.

100

118

Administration module

microbiome sample receiver

microbiome characterization platform

114

116

One or more hardware processors

110

108

Ensemble building module 120

Data Repository

112

I/O interface 104

102

**FIG. 1**

23

200

Collect a set of biological samples on a predefined set of days from the pregnant
subject whose EDD is to be estimated — 202

Perform microbiome profiling for each of the collected biological samples to obtain
a microbiome profile data comprising of a plurality of metagenomic features — 204

Predict a set of estimated delivery dates for the pregnant subject by providing the
microbiome profile data as input to a set of models present in a prebuilt ensemble — 206

Calculate a central tendency value of the predicted set of estimated delivery dates to
determine the EDD of the pregnant subject, and wherein the predefined set of days
are pre-ascertained — 208

**FIG. 2**

200

| Collect biological samples from a plurality of pregnant subjects at predefined time points during a period of pregnancy | 302 |

| Perform microbiome profiling for each of the collected biological samples to obtain a microbiome profile data comprising of the plurality of metagenomic features | 304 |

| Train one or a combination of regressors using the obtained microbiome profile data | 306 |

| Reconstruct using the trained regressors, for each pregnant subject among the plurality of pregnant subjects, a simulated microbiome profile, wherein the simulated microbiome profile comprises of values in original microbiome profile data & reconstructed values of all metagenomic features for specific days of pregnancy duration when samples were not collected from the pregnant subject | 308 |

| Construct using the simulated microbiome profiles, a multi-variate matrix for each of the metagenomic features, wherein the multi-variate matrix refers to the microbiome profile of the metagenomic feature observed in each of the pregnant subjects across entire duration of pregnancy, wherein each matrix comprises a participant ID corresponding to each pregnant subject in rows and each day of pregnancy, in which the microbiome profile of the metagenomic feature is observed, as columns | 310 |

| Train a regressor model with a k-fold cross validation on a predefined subset of rows in the multi-variate matrix for each metagenomic feature, wherein values of the metagenomic feature across the entire pregnancy duration belonging to the subset of the multi-variate matrix forms independent variables and corresponding date of delivery forms the target variable | 312 |

| Compute prediction errors of the regressor models generated during k-folds cross validation performed on the subset of the multi-variate matrix | 314 |

A          B

**FIG. 3A**

300

Ⓐ

Ⓑ

progressively lesser each pregnancy window corresponds to a pregnancy duration by one week as compared to the previous or the next pregnancy window
316

Compute corresponding prediction errors for input data corresponding to each pregnancy window
318

Identify for each metagenomic feature, a predefined number of top pregnancy windows which resulted in the smallest prediction errors
320

Select from amongst identified predefined number of top pregnancy windows, a pregnancy window that occurs the maximum number of times across all metagenomic features
322

Retrieve for each metagenomic feature, the respective regressor model corresponding to the selected pregnancy window the regressor model comprises a plurality of temporal features
324

Select another metagenomic feature in microbiome profile data
336

Sort the plurality of temporal features of the regressor model based on their importance scores and selecting, a set of top scoring temporal features, wherein the selected set of temporal features refer to a specific set of days within the selected pregnancy window
326

Identify and select, from amongst the days occurring in the selected pregnancy window, a predefined number of days that are associated to metagenomic features
328

Generate for each metagenomic feature, a feature-specific regressor model with a k-fold cross validation on a custom multi-variate matrix comprising of data pertaining to numeric values of the features on the identified specific subset of days
330

Select a subset of models out of the models trained, wherein the selected models have the lowest prediction error
332

Form an ensemble using the selected subset of models
334

**FIG. 3B**

Europäisches
Patentamt

European
Patent Office

Office européen
des brevets

# EUROPEAN SEARCH REPORT

**Application Number**

EP 22 17 6924

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2020/168118 A1 (MIRVIE INC [US]) 20 August 2020 (2020-08-20) * paragraph [0012] * * paragraph [0056] * * paragraph [0067] – paragraph [0070] * * paragraph [0128] * * paragraph [0130] – paragraph [0133] * * paragraph [0150] * * paragraph [0188] – paragraph [0190] * * paragraph [0263] – paragraph [0265] * * paragraph [0307] – paragraph [0308] * * claim 1 * | 1-13 | INV. G16H10/40 G01N33/48 G16H50/20 G16H50/70 |
| X | WO 2020/227053 A1 (UNIV VIRGINIA COMMONWEALTH [US]) 12 November 2020 (2020-11-12) * paragraph [0008] * * paragraph [0011] – paragraph [0013] * * paragraph [0018] * * paragraph [0025] – paragraph [0028] * * paragraph [0030] * * paragraph [0092] – paragraph [0093] * * claim 1 * | 1-13 | |
| X | WO 2019/084033 A1 (CHAN ZUCKERBERG BIOHUB INC [US] ET AL.) 2 May 2019 (2019-05-02) * claim 1 * * paragraph [0071] – paragraph [0072] * * paragraph [0084] * * paragraph [0088] * * paragraph [0147] – paragraph [0149] * | 1-13 | TECHNICAL FIELDS SEARCHED (IPC) G16H G01N |

-/--

The present search report has been drawn up for all claims

| Place of search | Date of completion of the search | Examiner |
|---|---|---|
| Munich | 20 October 2022 | Martínez Cebollada |

EPO FORM 1503 03.82 (P04C01)

page 1 of 2

Europäisches
Patentamt

European
Patent Office

Office européen
des brevets

# EUROPEAN SEARCH REPORT

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | FETTWEIS JENNIFER M ET AL: "The vaginal microbiome and preterm birth", NATURE MEDICINE, NATURE PUBLISHING GROUP US, NEW YORK, vol. 25, no. 6, 29 May 2019 (2019-05-29), pages 1012-1021, XP036901036, ISSN: 1078-8956, DOI: 10.1038/S41591-019-0450-2 [retrieved on 2019-05-29] * the whole document * | 1-13 | |
| A | WO 2020/037244 A1 (HENRY M JACKSON FOUND FOR THE ADVANCEMENT OF MILITARY MEDICINE [US] ET) 20 February 2020 (2020-02-20) * paragraph [0008] * * paragraph [0014] - paragraph [0016] * * paragraph [0021] - paragraph [0022] * | 1-13 | |

TECHNICAL FIELDS
SEARCHED      (IPC)

The present search report has been drawn up for all claims

| Place of search | Date of completion of the search | Examiner |
|---|---|---|
| Munich | 20 October 2022 | Martínez Cebollada |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 22 17 6924

This annex lists the patent family members relating to the patent documents cited in the above-mentioned European search report.
The members are as contained in the European Patent Office EDP file on
The European Patent Office is in no way liable for these particulars which are merely given for the purpose of information.

20-10-2022

| Patent document cited in search report | | Publication date | Patent family member(s) | | Publication date |
|---|---|---|---|---|---|
| WO 2020168118 | A1 | 20-08-2020 | AU | 2020221278 A1 | 05-08-2021 |
| | | | CA | 3126990 A1 | 20-08-2020 |
| | | | CN | 113692624 A | 23-11-2021 |
| | | | EP | 3924972 A1 | 22-12-2021 |
| | | | GB | 2597379 A | 26-01-2022 |
| | | | JP | 2022519897 A | 25-03-2022 |
| | | | SG | 11202108405V A | 30-08-2021 |
| | | | US | 2021017598 A1 | 21-01-2021 |
| | | | US | 2022073982 A1 | 10-03-2022 |
| | | | WO | 2020168118 A1 | 20-08-2020 |
| WO 2020227053 | A1 | 12-11-2020 | US | 2022243247 A1 | 04-08-2022 |
| | | | WO | 2020227053 A1 | 12-11-2020 |
| WO 2019084033 | A1 | 02-05-2019 | CN | 111566228 A | 21-08-2020 |
| | | | EP | 3701043 A1 | 02-09-2020 |
| | | | JP | 2021500061 A | 07-01-2021 |
| | | | WO | 2019084033 A1 | 02-05-2019 |
| WO 2020037244 | A1 | 20-02-2020 | US | 2021327540 A1 | 21-10-2021 |
| | | | WO | 2020037244 A1 | 20-02-2020 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- IN 202121024679

**Non-patent literature cited in the description**

- **FETTWEIS, J.M. ; SERRANO, M.G. ; BROOKS, J.P. et al.** The vaginal microbiome and preterm birth. *Nat Med,* 2019, vol. 25, 1012-1021